# EUROPEAN PATENT APPLICATION

(11) **EP 1 371 383 A1**
(43) Date of publication of application: **17.12.2003**
(21) Application number: 03013333.4
(22) Date of filing: 13.06.2003
(51) Int. Cl.: A61M 5/32

(54) **Needle shield assembly**

(30) Priority: 14.06.2002 US 173230
(71) Applicant: Becton, Dickinson and Company, Franklin Lakes, New Jersey 07417-1880 (US)
(72) Inventor: Crawford, Jamieson W.M., New York, New York 10025 (US); Swenson, Kirk D., North Caldwell, New Jersey 07006 (US); Newby, C. Mark, Tuxedo, New York 10987 (US); Hwang, Charles G., Ridgewood, New Jersey 07450 (US)
(74) Representative: Müller-Boré & Partner Patentanwälte

(57) **Abstract**

A medical implement is provided with a hub (60) and a piercing element (40) that projects distally from the hub. A protective cap (52) is removably engaged with the proximal end of the hub. An IV shield (50) is threadedly engaged with the distal end of the hub and protectively covers the piercing element (40). A hinged shield (140) is hingedly mounted to the hub and can be rotated from a first position substantially adjacent the IV shield to a second position where the hinged shield is spaced rotationally from the IV shield and finally to a third position where the hinged shield encloses the piercing element. The hinged shield must be rotated from the first position to the second position to threadedly disengage the IV shield and to expose the piercing element for use. After use, the hinged shield is rotated from the second position into the third position for protectively enclosing the piercing element.

## Description

### 1. Field of the Invention

The present invention relates to a shield for a needle and more particularly to a safety shield assembly that may be used in conjunction with a syringe assembly, a hypodermic needle, a needle assembly, a needle assembly with a needle holder, a blood collection needle, a blood collection set, an intravenous infusion set or other fluid handing devices or assemblies that contain piercing elements.

### 2. Background of the Invention

Disposable medical devices having piercing elements for administering a medication or withdrawing a fluid, such as hypodermic needles, blood collecting needles, fluid handling needles and assemblies thereof, require safe and convenient handling. The piercing elements include, for example, pointed needle cannula or blunt ended cannula. The piercing element typically is mounted to a hub and extends distally from the hub. A second piercing element may extend proximally from the hub, and may include a non-patient needle that can be placed in communication with a container, such as an evacuated fluid collection container. In other instances, the proximal end of the hub is configured for mating with a medical implement, such as a syringe or a needle holder.

Safe and convenient handling of disposable medical devices is recognized by those in the medical arts so as to minimize exposure to blood borne pathogens. Safe and convenient handling of disposable medical devices results in the disposal of the medical devices intact.

As a result of this recognition, numerous devices have been developed for shielding needles after use. Many of these devices are somewhat complex and costly. In addition, many of these devices are cumbersome to use in performing procedures. Furthermore, some of the devices are so specific that they preclude use of the device in certain procedures or with certain devices and/or assemblies. For example, some devices employ very short thin needle cannulas. A shield designed to lock near the distal end of one needle cannula might not engage a much shorter needle cannula. Additionally, a shield designed to lock with a wider gauge needle cannula might be more likely to generate a spray upon engaging a much narrower needle cannula. Furthermore, it may be desirable to reduce the force required to effect shielding without reducing the audible and tactile indications of complete shielding.

Some medical devices employ a plurality of shields, sleeves and/or caps to achieve sterility and to prevent accidental needle sticks prior to use and to further prevent accidental needle sticks after use. For example, some medical devices employ a rigid generally tubular cap or sleeve telescoped over the piercing element that projects from the distal end of the hub. A second cap or sleeve is telescoped into or over the proximal end of the hub to provide sterility and to prevent accidental sticks with any piercing element that projects from the proximal end of the hub. A hinged shield may be provided on the medical device to prevent accidental sticks with at least the distally directed piercing element after use of the medical device.

Manufacturers of medical devices have preferred methods of use to optimize safe handling, to ensure maximum cleanliness and to avoid accidental sticks. In particular, the user is instructed to remove the proximal cap or sleeve from the hub before removing the sleeve over the piercing element at the distal end of the hub and before manipulating any hinged shield that may be mounted to the hub. The proximal end of the hub then is mounted to the medical device with the distal sleeve in place over the piercing element that projects distally from the hub. Any hinged shield that may be provided on the device then is rotated into a position away from the piercing element, while still keeping the distal sleeve telescoped over the piercing element. The distal sleeve is removed immediately prior to use and is discarded. The medical device then is employed in a specified safe manner. After use, the hinged shield or other such post-use shielding element is moved into a position surrounding the piercing element, and at least portions of the medical device are disposed of in a safe manner.

A need exists for a safety shield assembly: (i) that is manufactured easily; (ii) that is applicable to many devices; (iii) that is simple to use with one hand; (iv) that can be disposed of safely; (v) that does not interfere with normal practices of needle use; (vi) that has tactile features whereby the user may be deterred from contacting the needle, the user may easily orient the needle with the patient and easily actuate and engage the shield assembly; (vii) that has visual features whereby the user may be deterred from contacting the needle, the user may easily orient the needle with the patient and easily actuate and engage the shield assembly; (viii) that is not bulky; (ix) that includes means for minimizing exposure to the user of residual fluid leaking from the needle; and (x) provides minimal exposure to the user because the needle shield is immediately initiated by the user after the needle is withdrawn from the patient's vein. It also would be desirable to provide a safety shield assembly with greater assurance that the preferred unshielding and shielding steps are carried out in a specified safe sequence.

### 3. Summary of the Invention

The invention relates to a fluid handling device with opposite proximal and distal ends. A piercing element projects at the distal end of the fluid handling device. The piercing element at the distal end of the fluid handling device may comprise a metallic needle cannula, a plastic cannula, a blunt cannula or other piercing element for delivering a fluid to a patient or for obtaining a specimen of fluid. The proximal end of the fluid handling device is configured for communication with another medical implement. For example, the proximal end of the fluid handling device may be configured for mating to a syringe, a fitting on a fluid transfer line, a holder for receiving an evacuated tube or other known medical implement for delivering a fluid to a patient or for obtaining a sample of fluid. In particular, the proximal end of the fluid handling device may define a female Luer fitting. Alternatively, the proximal end of the fluid handling device may comprise a proximal piercing element, such as a non-patient needle intended for communication with an evacuated tube or other medical device.

The fluid handling device further comprises a cap or proximal sleeve mounted over at least portions of the proximal end of the fluid handling device. The cap or proximal sleeve mounted to the proximal end of the fluid handling device has a configuration dependent on the specific structure provided at the proximal end of the fluid handling device. For example, a tubular proximal sleeve may be mounted to the proximal end of the fluid handling device for those instances where the fluid handling device includes a proximal piercing element. Alternatively, an end cap may be mounted to the proximal end of the fluid handling device for those instances where the fluid handling device is configured for mating with a syringe, a fitting or other medical implement. The proximal sleeve or end cap may be frictionally or threadedly mounted to proximal portions of the fluid handling device.

A distal sleeve is telescoped removably to distal portions of the fluid handling device and is configured for protectively covering the piercing element. Proximal portions of the distal sleeve preferably are connected threadedly to the fluid handling device. For example, proximal portions of the distal sleeve may be formed with an array of external threads that can threadedly engage an array of internal threads at or near proximal portions of the distal piercing element. Alternatively, proximal portions of the distal sleeve may include an array of internal threads that engage external threads on the fluid handling device.

The fluid handling device further comprises a hinged safety shield that is intended for shielding the distal piercing element after use. The hinged shield is preliminarily mounted in a position partly surrounding the distal sleeve. However, the dimensions of the distal sleeve prevent the hinged shield from rotating completely over the distal sleeve. The hinged shield is intended to be rotated away from the distal sleeve and away from the piercing element covered by the distal sleeve prior to use of a fluid handling device. After use, the hinged shield is rotated toward the distal piercing element and locks into engagement around the distal piercing element, as explained further herein.

The initial position of the hinged shield in partly surrounding relationship to the distal sleeve prevents or complicates any attempt to threadedly disengage the distal sleeve from the fluid handling device. Hence, the hinged shield must be rotated into the ready-to-use position prior to removal of the distal sleeve. Thus, the fluid handling device inherently ensures that the user of the fluid handling device will follow the preferred safe sequence of first rotating the hinged shield into the ready-to-use position and then removing the distal sleeve. The user is substantially prevented from following the less safe sequence of first removing the distal sleeve and then rotating the hinged shield while the distal piercing element is exposed.

The hinged shield may take many forms. Preferably, the hinged shield comprises a rearward end, a forward end, a slot or longitudinal opening for housing the used needle in the forward end, means for securing the needle in the slot, means for guiding the needle into the slot, means for connecting the hinged shield and the fluid handling device, means for guiding the user's fingers to move the hinged shield into various positions, and means for retaining the hinged shield securely over the used needle.

Desirably, the means for connecting the hinged shield to the fluid handling device is a collar. Preferably, the hinged shield is connected movably to a collar which is connected to a fluid handling device.

Preferably, the hinged shield is connected to the collar by a hanger bar that engages with a hook arm on the collar so that the hinged shield may be pivoted with respect to the collar into several positions. It is within the purview of the present invention to include any structure for connecting the hinged shield to the collar so that the shield may be pivoted with respect to the collar. These structures include known mechanical hinges and various linkages, living hinges, or combinations of hinges and linkages.

Most preferably, the hinged shield is connected to the collar by an interference fit between the hanger bar and the hook bar. Therefore, the shield always is oriented in a stable position and will not move forward or backwards unless movement of the hinged shield relative to the hanger bar and the hook bar is initiated by the user.

Alternatively, the hinged shield and collar may be a unitary one-piece structure. The one-piece structure may be obtained by many methods, including molding the shield and the collar as a one-piece unit, thereby eliminating the separate shield and collar during the manufacturing assembly process.

The assembly of the present invention may further comprise tactile and visual means for deterring the user from contacting the needle, providing easy orientation of the needle with the patient and providing the user with a guide for actuation and engagement with the hinged shield.

The assembly of the present invention may further comprise means for minimizing exposure by the user to residual fluid leaking from a used needle. For example, a polymer material, such as a gel, may be located in the hinged shield.

Most desirably, the assembly of the present invention is such that the cooperating parts of the assembly provide the means for the hinged shield to move into a forward position over the needle. Thus, by simple movement of the hinged shield into a forward position over the used needle, the assembly is ready for subsequent disposal. Therefore, the assembly of the present invention provides minimal exposure of the user to a needle because the shielding is initiated by the user immediately after the needle is withdrawn from the patient's vein.

Desirably, the assembly of the present invention may be used with a syringe assembly, a hypodermic needle, a needle assembly, a needle assembly with a needle holder, a blood collection set, an intravenous infusion set or other fluid handling devices. Preferably, the assembly of the present invention is used with a needle assembly comprising a needle and a hub. Preferably the needle is a conventional double ended needle.

Most preferably, the present invention is used with a needle assembly comprising a hub and a needle connected to the hub whereby the needle comprises a non-patient end and an intravenous end. The collar of the present invention may comprise a hook arm and the hinged shield may be connected movably to the hook arm. Thus the hinged shield may be positioned with respect to the collar and moved easily into several positions.

Preferably, the collar is fitted non-rotatably with the hub of the needle assembly. Additionally, the collar includes cooperating means that mate with reciprocal means on the shield to provide a clear audible and tactile indication of shielding. The cooperating means on the collar may include generally chevron-shaped projection formed on a side of the collar substantially diametrically opposite the hook arm or other such structure that provides the hinge connection to the shield. The chevron-shaped structure includes a forward or distal point. Slanting surfaces diverge and extend proximally from the distal point. The slanting surfaces cooperate with the reciprocal means on the shield to generate a deflection of the sidewalls of the shield away from one another. The chevron-shaped structure further includes proximal ends that are convexly arcuate. The convexly arcuate ends of the chevron-shaped structure on the collar cooperate with the reciprocal means on the shield and with the resiliently deflectable sidewalls of the shield to generate the tactile and audible indication of shielding.

The hinged shield preferably includes at least one cannula finger lock for locked engagement with the cannula when the hinged shield is in the second position around the needle cannula. The cannula finger lock preferably projects obliquely from one sidewall of the hinged shield angularly toward the opposed sidewall and the top wall of the shield. The cannula finger lock is dimensioned, disposed and aligned to contact the needle cannula when the hinged shield approaches the second position. Contact between the cannula and the cannula finger lock will cause the cannula finger lock to resiliently deflect toward the sidewall from which the cannula finger lock extends. Sufficient rotation of the hinged shield will cause the needle cannula to pass the cannula finger lock. As a result, the cannula finger lock will resiliently return to or toward its undeflected condition for securely trapping the needle cannula in the hinged shield.

Preferably, the collar is fitted with the hub of the needle assembly so that the collar cannot rotate around the hub.

Alternatively, the collar and hub may be a unitary one-piece structure. The one piece structure may be accomplished by many methods including molding the collar and the hub as a one-piece unit thereby eliminating the need to separately assemble the collar to the hub during the manufacturing process.

Most preferably, the collar is fitted with the hub of the needle assembly so that the bevel surface or bevel up surface of the intravenous or distal end of the needle faces the same side of the collar when the hinged shield is in the open position. Alignment of the collar, hub, hinged shield and needle with the bevel surface up makes it easier to insert the needle into the patient without manipulating the assembly. The orientation of the intravenous end of the needle with the bevel up assures the user that the needle is properly oriented for use and does not require any manipulation before use. Most notably, the orientation of the hinged shield provides a visual indication to the user of the orientation of the bevel surface of the needle.

Preferably, the hinged shield is capable of pivoting from a first position, where the intravenous end of the needle is exposed and bevel up, to an intermediate position where the needle is partially covered, to a second position where the needle is contained by the shield.

Alternatively, it is within the purview of the present invention that the hinged shield, collar and hub is a unitary one-piece structure. The one-piece structure may be accomplished by many methods including molding the hinged shield, collar and hub as a one-piece unit thereby eliminating the need to separately assemble the hinged shield, collar and hub during the manufacturing process.

It is an advantage of the present invention that the hinged shield covering the used intravenous end of the needle provides easy containment of the used needle. A further advantage of the hinged shield is that it will only move upon initiation by the user.

The assembly of the present invention when used with a fluid handling device is also easily disposable when removed from a conventional needle holder, or other such device.

A notable attribute of the present invention is that it is easily adaptable with many devices. For example, the invention is usable with syringe assemblies, hypodermic needles, needle holders, blood collection needles, blood collection sets, intravenous infusion sets such as catheters or other fluid handling devices or assemblies that contain piercing elements.

Another notable attribute of the present invention is that the tactile and visual features deter the user from touching the needle, allow the user to easily orient the needle with the patient and guide the user to actuate and engage the shield of the assembly.

### 4. Brief Description of the Drawings

FIG. 1 is a perspective view of the safety shield assembly of the present invention as connected to a needle assembly and related packaging features.

FIG. 2 is a perspective view of the unassembled pieces of FIG. 1.

FIGS. 3A and 3B are bottom views of the shield as shown in FIG. 2.

FIG. 4 is a cross sectional view of the collar as shown in of FIG. 2 taken along lines 4-4 thereof.

FIG. 5 is a cross sectional view of the needle hub as shown in FIG. 2 taken along lines 5-5 thereof.

FIG. 6 is a cross sectional view of the shield of FIG. 2 taken along lines 6-6 thereof.

FIGS. 7-12 illustrate the use of the safety shield assembly with the needle assembly of FIG. 1 with a conventional needle holder.

FIG. 13 is a cross sectional view of the assemblies in use with a conventional needle holder as shown in FIG. 12 taken along lines 13-13 thereof.

FIG. 14 is a cross-sectional view of the assemblies of FIG. 13 taken along lines 14-14 thereof.

FIG. 15 is a bottom view of the assemblies as shown in FIG. 11.

FIG. 16 illustrates an additional embodiment of the present invention, whereby a gel material is located in the shield as shown in a bottom view of the assemblies of FIG. 11.

FIG. 17 is a perspective view of an additional embodiment of the present invention in use with a blood collection set.

FIG. 18A is an exploded perspective view of an additional embodiment of the present invention intended for use with a syringe.

FIG. 18B is a perspective view of the collar of the embodiment of FIG. 18A.

FIG. 18C is a side elevational view of the embodiment of FIG. 18A mounted to a syringe.

FIG. 19 is a perspective view of an additional embodiment of the present invention in use with a catheter.

### 5. Detailed Description of the Invention

While this invention is satisfied by embodiments in many different forms, there is shown in the drawings and will herein be described in detail, the preferred embodiments of the invention, with the understanding that the present disclosure is to be considered as exemplary of the principles of the invention and is not intended to limit the invention to the embodiments illustrated. Various other modifications will be apparent to and readily made by those skilled in the art without departing from the scope and spirit of the invention. The scope of the invention will be measured by the appended claims and their equivalents.

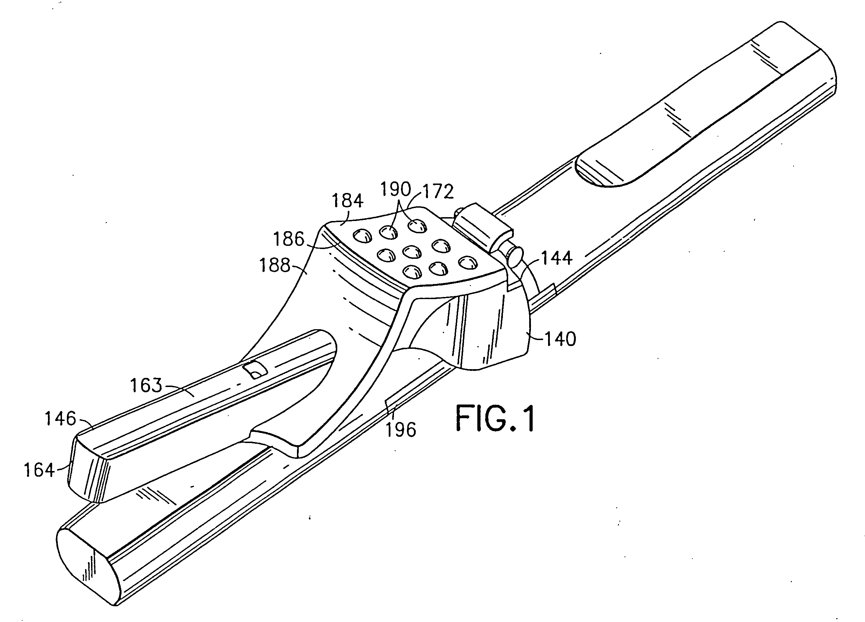

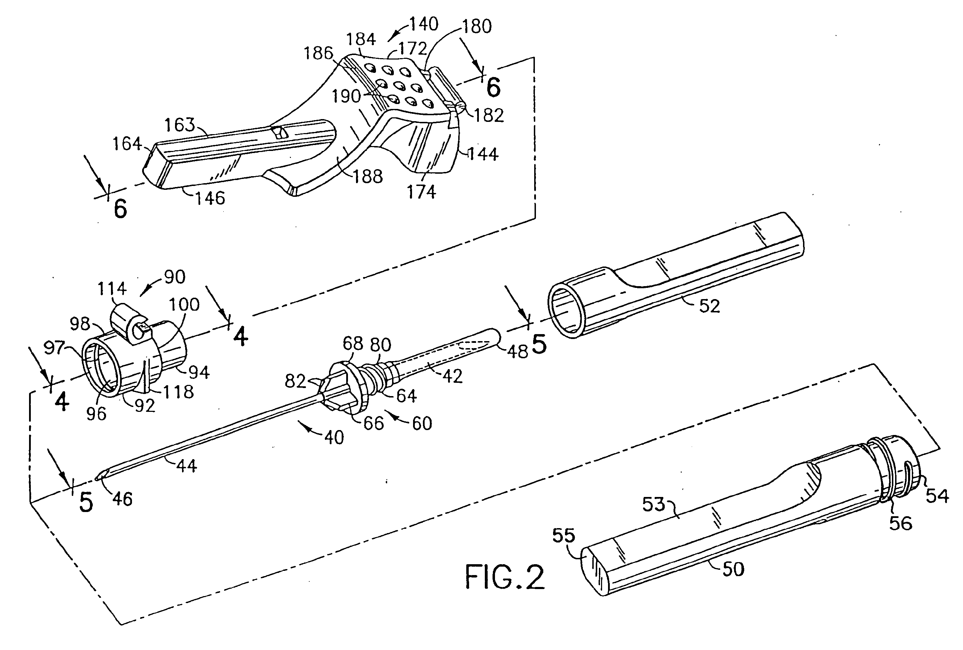

Referring to the drawings in which like reference characters refer to like parts throughout the several views thereof, FIGS. 1 and 2 illustrate a needle assembly with the safety shield assembly of the present invention and the related packaging features. The needle assembly includes a needle **40**, a hub **60**, packaging features to cover the needle and a label. The safety shield assembly includes a collar **90** and a hinged shield **140**.

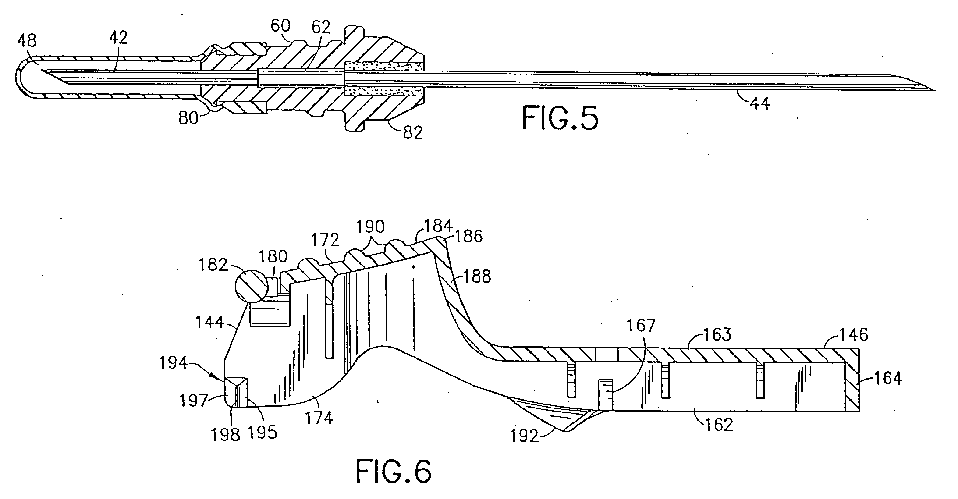

As shown in FIG. 2 and 5, needle **40** includes a non-patient end **42**, an intravenous end **44** and a passageway **46** extending between the non-patient end and the intravenous end. An elastomeric sleeve **48** covers the non-patient end. A first rigid sleeve **50** covers the intravenous end and a second rigid sleeve **52** covers both the non-patient end and the elastomeric sleeve. As shown in FIG. 1, a label **196** may also be applied to the finally assembled parts. First rigid sleeve **50** includes a rigid tubular sidewall **53** with an open proximal end **54** and a closed distal end **55**. An array of external threads **56** extends around first rigid sleeve **50** adjacent proximal end **54**.

As shown in FIGS. 2 and 5, hub **60** includes a threaded end **64**, a ribbed end **66** and passageway **62** extending between the threaded end and the ribbed end. Threaded end **64** and ribbed end **66** are separated by flange **68**. Non-patient end **42** of needle **40** extends from threaded end **64** and intravenous end **44** of needle **40** extends from ribbed end **66**. Preferably, threaded end **64** comprises male threads **80** for mounting the hub on a conventional needle holder and ribbed end **66** comprises male ribs **82** for connecting the hub and collar **90**.

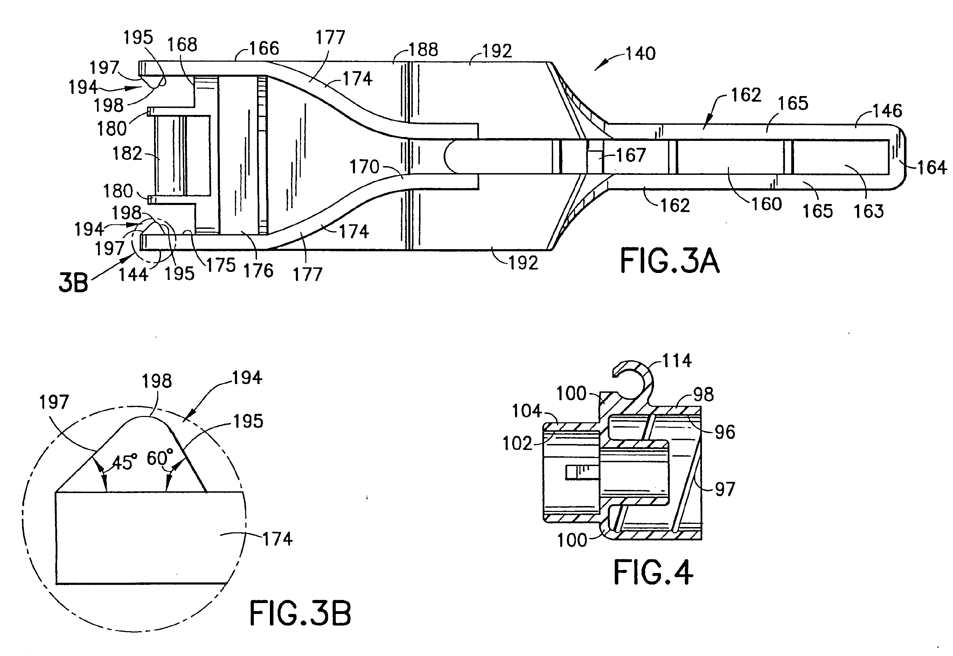

As shown in FIGS. 2 and 4, collar **90** includes a forward skirt **92** and a rearward skirt **94**. Forward skirt **92** is cylindrical and comprises an inner circumferential surface **96** with an array of internal threads **97** and an outer circumferential surface **98**. Forward skirt **92** mates with rearward skirt **94** at a shoulder **100**. Rearward skirt **94** is cylindrical and comprises an inner circumferential surface **102** and an outer circumferential surface **104** and extends from shoulder **100** opposite of forward skirt **92**. The inner diameter of forward skirt **92** is larger than the inner diameter of rearward skirt **94**. Alternatively, the inner diameters for collar **90** can be equal. A hook **114** extends from outer circumferential surface **98** of forward skirt **92**. Additionally a chevron-shaped protrusion **118** projects outwardly from outer circumferential surface **98** of forward skirt **92** at a side opposite hook **114**. The chevron-shape protrusion **118** is substantially symmetrically formed and has a peak **120** pointed toward forward skirt **92** and ramp surfaces **122** that diverge symmetrically from peak **120** toward rearward skirt **94**. Ramp surfaces **122** terminate at rounded ends **124** at the outer side and proximal extremes of chevron-shaped protrusion **118**. Rounded ends **124** extend continuously into the proximal side of chevron-shaped protrusion **118** facing toward rearward skirt **94**.

As shown in FIGS. 2 and 6, hinged shield **140** comprises a rearward end **144** and a forward end **146**.

Forward end **146** of hinged shield **140** includes a slot or longitudinal opening **160** formed by sidewalls **162** that extend downwardly from top wall **163** and run substantially opposite of one another in parallel along the length of slot **160** towards forward end wall **164**. Slot **160** is slightly wider than needle **40**. Sidewalls **162** include bottom edges **165** that extend substantially parallel to one another and parallel to top wall **163**.

A cannula finger lock **167** is located at one of sidewalls **162** and is configured to secure the used needle. Cannula finger lock **167** extends from a location on a first of the sidewalls **162** adjacent the bottom edge **165** thereof and projects angularly toward the opposed sidewall **162** and toward the top wall **163**. The projection of the cannula finger lock **167** from the respective sidewall **162** preferably exceeds half the distance between the respective sidewalls. Cannula finger lock **167** is deflectable by the needle when the needle enters slot **160**. Once the needle passes the end of cannula finger lock **167**, the cannula finger lock moves back to its original position so that the needle is permanently trapped in slot **160** by cannula finger lock **167**.

Rearward end **144** of hinged shield **140** defines a collar engaging area **166** that is a continuation of slot **160**. Collar engaging area **166** includes a rearward end **168**, a forward end **170**, a top finger guide area **172**, sidewalls **174** that extend downwardly from top finger guide area **172**, an underside area **176** dimensioned for surrounding collar **90**, and extending arms **180** to support and hold hanger bar **182**. Sidewalls **174** are spaced apart by a major width adjacent rearward end **168**. The major width is selected to enable sidewalls **174** to slide across diametrically opposite side surfaces of forward skirt **92** of collar **90**. Sidewalls **174** converge, however, toward forward end **170** to define a minor distance therebetween substantially equal to the distance between sidewalls **162** at forward end **146** of hinged shield **140**. Sidewalls **174** include bottom edges **177** that face away from top finger guide area **172**. As shown most clearly in FIG. 6, bottom edges **177** curve toward top finger guide area **172** at locations between rearward end **168** and forward end **170** of collar engaging area **166**.

The extreme rear ends of sidewalls **174** on collar engaging area **166** include rounded ears **194** that project toward one another from opposed inner surfaces **175** of sidewalls **174**. Rounded ears **194** are disposed to engage chevron-shaped protrusion **118** on collar **90**. More particularly, each rounded ear **194** includes a distal surface **195**, a proximal surface **197** and a curved surface **198** extending between distal and proximal surfaces **195** and **197**. Distal surface **194** is aligned to sidewall **174** at an angle of approximately 60° and proximal surface **197** is aligned to sidewall **174** at an angle of approximately 45°. Curved surface **198** extends smoothly and convexly between distal and proximal surfaces **195** and **197**. Proximal surfaces **197** of rounded ears **194** will engage ramp surfaces **122** of chevron-shaped protrusion **118** to deflect sidewalls **174** slightly away from one another as hinged shield **140** approaches the second position. This deflection of sidewalls **174** will occur substantially simultaneously with the deflection of cannula finger lock **167**. The apex of curved surface **198** on each rounded ear **194** passes the respective rounded proximal end surface **124** on chevron-shaped projection **118** on collar **90** slightly before cannula finger lock **167** passes the needle cannula. As a result, sidewalls **174** begin to return resiliently toward an undeflected condition. This resilient return of sidewalls **174** cooperates with raked distal surfaces **195** on rounded ears **194** to cause sidewalls **174** to snap against chevron-shaped projection **118**. This snapping action provides a clear audible and tactile indication of complete shielding and occurs substantially when the used needle is trapped by cannula finger lock **167**. The angles of distal and proximal surfaces **195** and **197** of rounded ears **194** affects the performance of hinged shield **140**. In particular, a smaller acute angle alignment of proximal face **197** reduces the force required to move hinged shield **140** past rounded ears **194**. A larger acute angle proximal surface **197** of rounded ears **194** requires a greater force to move hinged shield **140** toward the second position. Similarly, the angle between distal surface **195** and sidewall **174** affects the acceleration characteristics as hinged shield **140** is propelled toward the second position in response to the resilient return of sidewalls **174**. This change in acceleration characteristics affects the audible indication of shielding.

Top finger guide area **172** comprises a first ramp **184** that extends slightly on an upwardly slope from the rearward end of the collar engaging area to a shoulder **186**. From shoulder **186** extends a second ramp **188** which slopes downwardly towards top section **163**. Most preferably, first ramp **184** comprises touch bumps **190**. The touch bumps provide a tactile and visual guide to alert the user that the user's finger has contacted the shield and that the shield is in a defined or controlled position. The touch bumps may be any configuration so long as they extend and are distinct from the top finger guide area. The touch bumps may also be of a distinguishing color as compared to the top finger guide area or the shield.

Second ramp **188** has interior surface **192** for urging the needle toward the center of slot **160** as the shield is being rotated into the closed position. The exterior surfaces are slightly inclined and extending radially from the second ramp. The interior surfaces are especially helpful if the longitudinal axis of the needle is misaligned with respect to the longitudinal axis of the hub.

Extending arms **180** are located at rearward end **168** and at the beginning of top finger area **172** and hold hanger bar **182**.

The safety shield assembly and the needle assembly are assembled together whereby needle **40** is connected to hub **60** and sealed with adhesive at the ends of the hub. Hub **60** then is joined with collar **90** by ultra-sonic welding techniques or any other bonding techniques, or mechanical fit, whereby rearward annular skirt **94** of collar **90** mates with ribbed end **66** of the hub. Male ribs **82** of the hub are contained or forced fitted within inner sidewall **102** of rearward annular skirt **94** of collar **90**. Collar **90** is aligned with the intravenous end of needle **40** whereby the hook **114** is aligned with the bevel up of needle **40**. External threads **96** adjacent proximal end **54** of first rigid sleeve **50** then are threaded into engagement with internal threads **97** formed on inner circumferential surface **96** of forward skirt **92** of collar **90** to cover needle **40**. Thereafter, hinged shield **140** is connected to collar **90** whereby hanger bar **182** is force fitted into hook **114** whereby slot **160** faces first rigid sleeve **50**. Most preferably, hinged shield **140** is connected to the collar by a force fit or interference fit between hanger bar **182** and hook **114**. Therefore, hinged shield **140** is always oriented in a stable position and will not move unless movement of the shield is positively initiated by the user. To assemble the last piece, shield **140** is moved towards rigid sleeve **50** and second rigid sleeve **52** is force fitted onto outer sidewall **104** of rearward skirt **94** of collar **90**.

In addition, a label **196** may be applied to the finally assembled parts. The label may be used to provide tamper resistance of the parts, so that they are not reused.

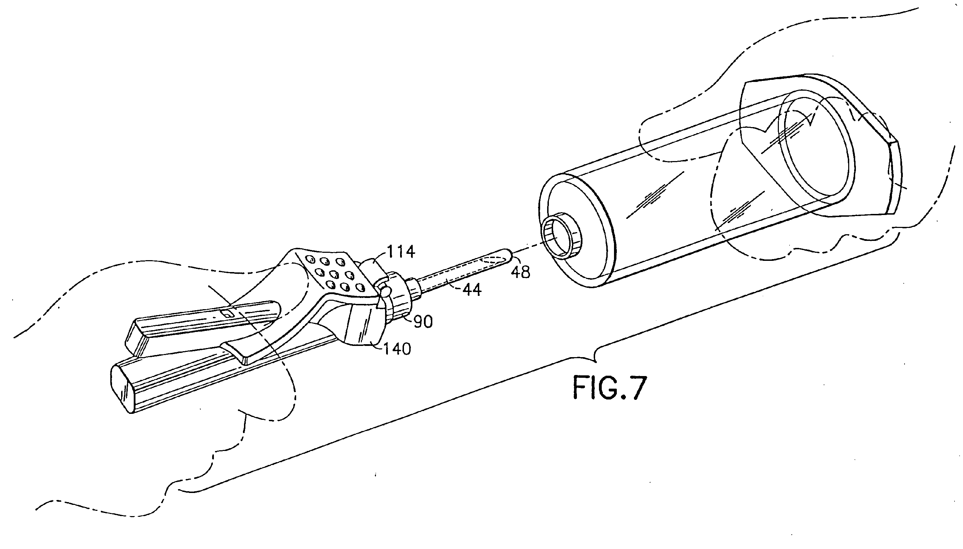

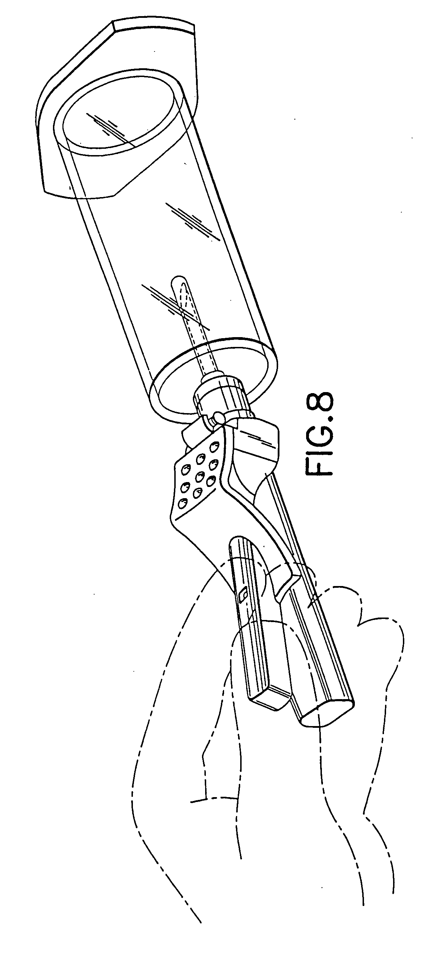

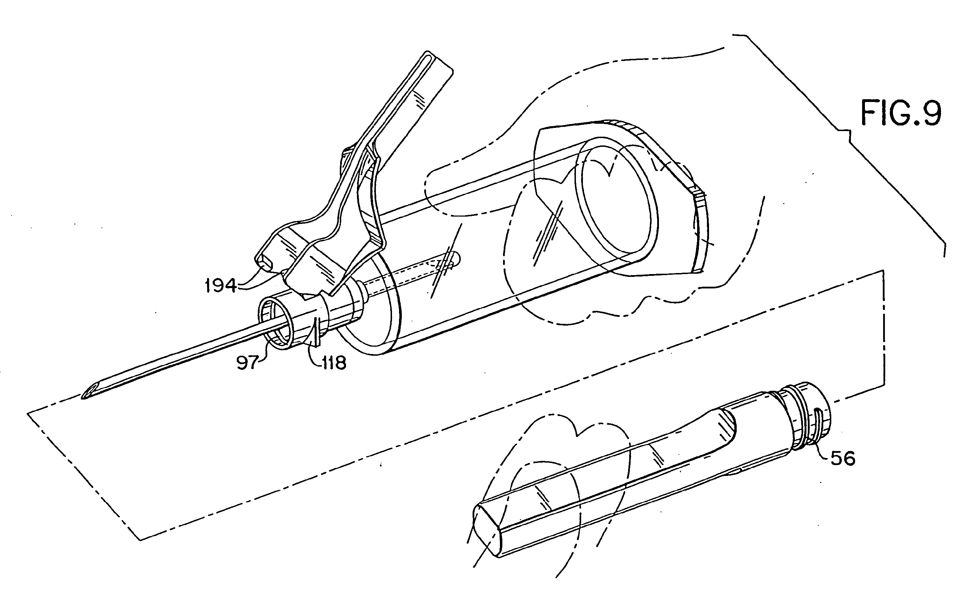

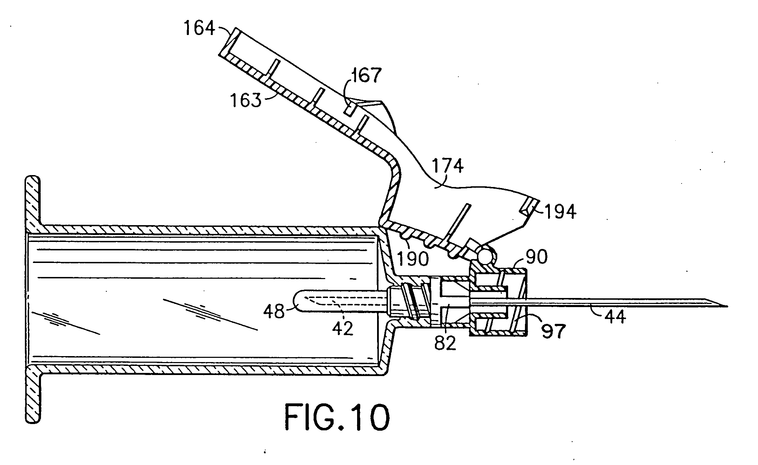

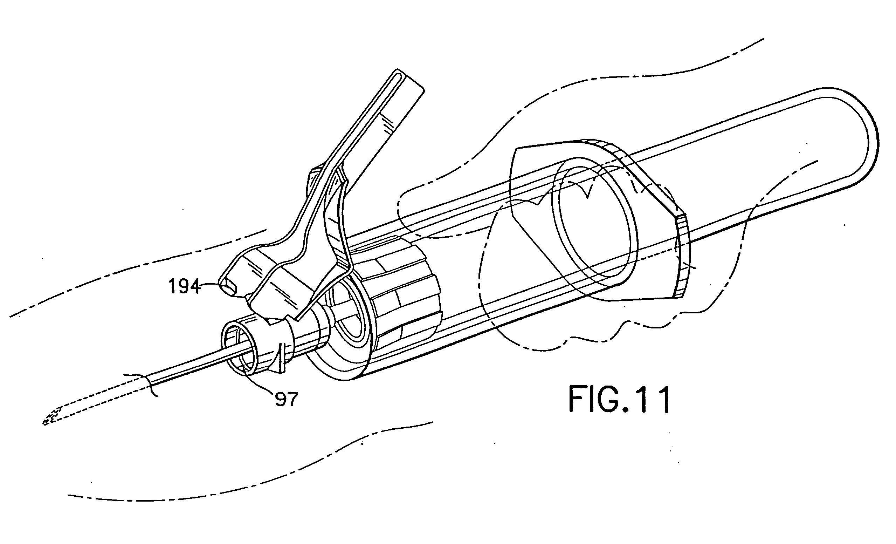

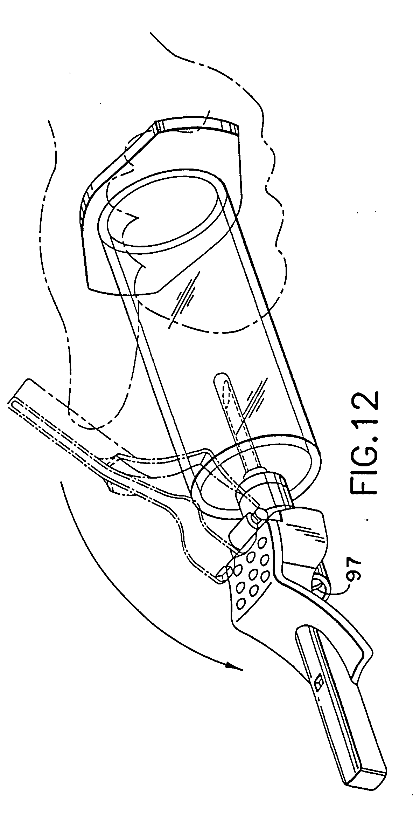

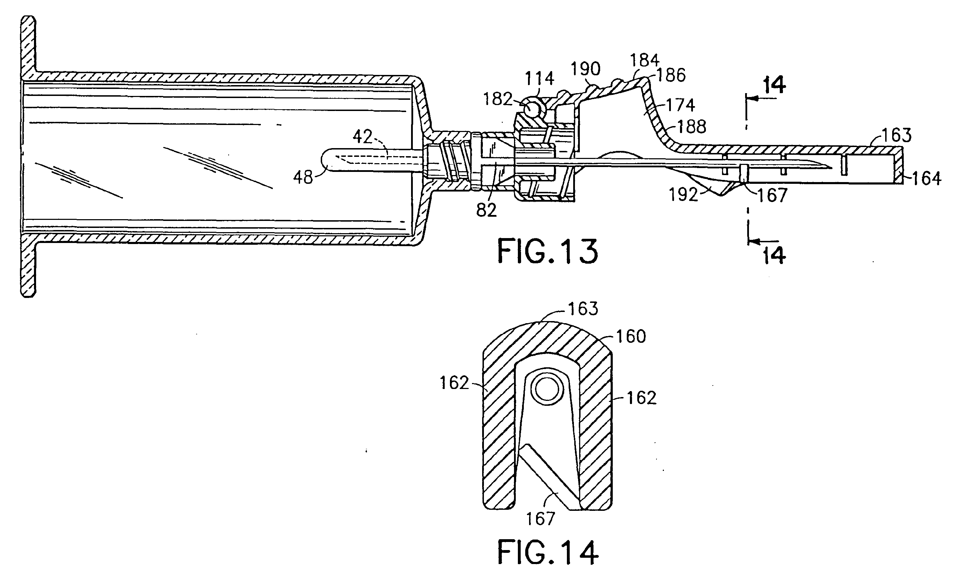

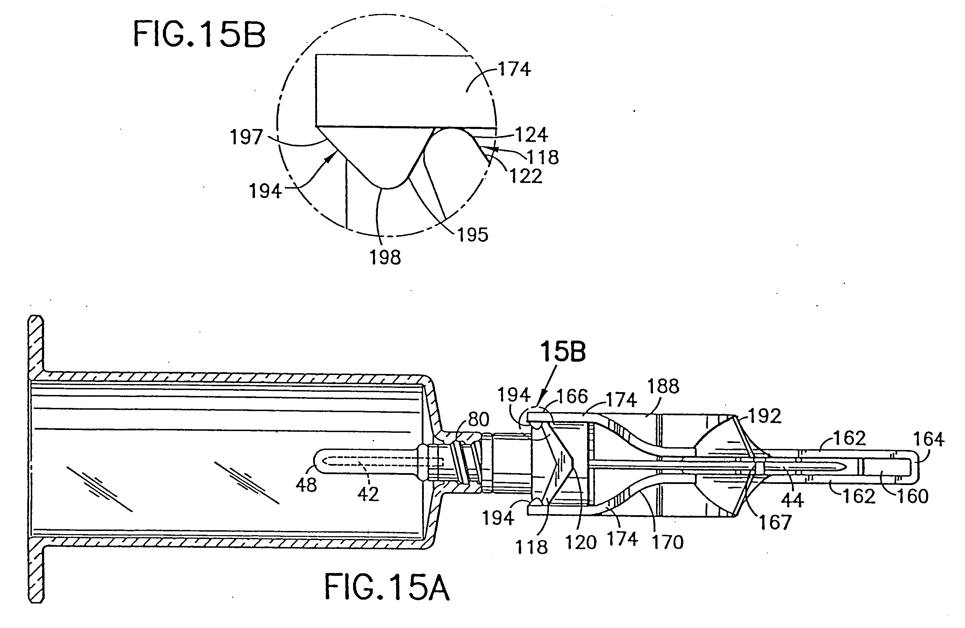

In use, as shown in FIGS. 7-15, second rigid sleeve **52** is removed from the non-patient needle by pulling proximally on second rigid sleeve **52**. A slight twisting force may be required to tear label **196**. A needle holder then is screwed onto threads **64** of hub **60**. As specifically shown in FIGS. 9 and 10, hinged shield **140** then is rotated back by the user towards the needle holder and first rigid sleeve **50** is threadedly disengaged from forward skirt **92** of collar **90** to remove the covering from the intravenous needle. Then as shown in FIG. 11, a venipuncture is conducted whereby the intravenous end of the needle is inserted into a vein of a patient and an evacuated tube having a closure is inserted into the needle holder. Then as shown in FIGS. 12-15, when the venipuncture is complete the user easily rotates hinged shield **140** from the open position towards the intravenous needle to an intermediate position and then the user pushes on the shield at the top finger guide area to move the shield into a second position whereby the needle is trapped in the longitudinal opening. More particularly, needle **40** contacts cannula finger lock **167**. The engagement of needle **40** with cannula finger lock **167** causes cannula finger lock **167** to deflect toward top wall and toward the sidewall **162** from which cannula finger lock **167** projects. Sufficient rotation of hinged shield **140** will cause needle **40** to pass cannula finger lock **167**. As a result, cannula finger lock **167** will return resiliently to an undeflected condition. Thus, needle **40** will be trapped above cannula finger lock **167**.

Needle **44** is contained within hinged shield **140** as the shield is pivoted into the second position. More particularly, proximal surfaces **197** of rounded ears **194** move over detents **118** and cause sidewalls **174** to deflect away from one another. The angularly aligned proximal faces **197** of rounded ears **194** ensure easy movement of shield **140**. Additionally, the resiliency of sidewalls **174** and the angular alignment of distal surface **195** of ears **194** causes hinged shield **140** to be accelerated into the second position. This accelerated movement of shield **140** helps to generate a clear audible and tactile indication of shielding.

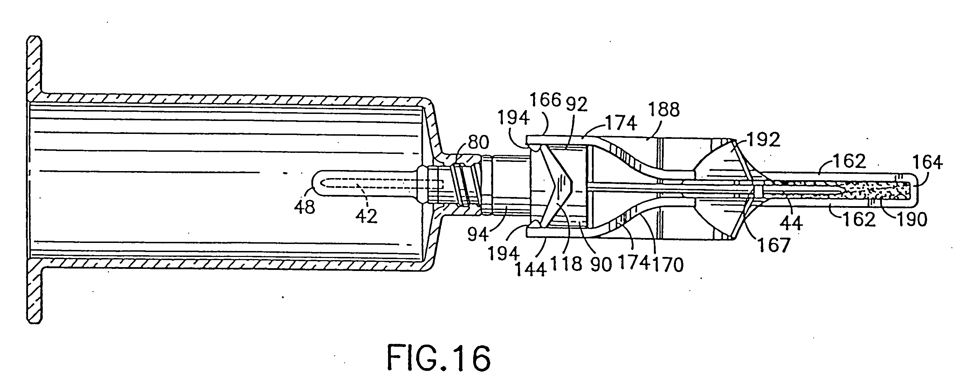

Alternatively as shown in FIG. 16, a gel material **190** is located in hinged shield **140** so that when the needle snaps past cannula finger lock **167** it will come to rest in gel material **190**. The gel material will contain any residual fluid that may be on the needle. Simultaneously, rounded ears or projections **198** move over detents **118**. This causes sidewalls **174** to deflect away from one another and then to snap back into engagement with collar **90** to provide a clear audible and tactile indication of complete shielding.

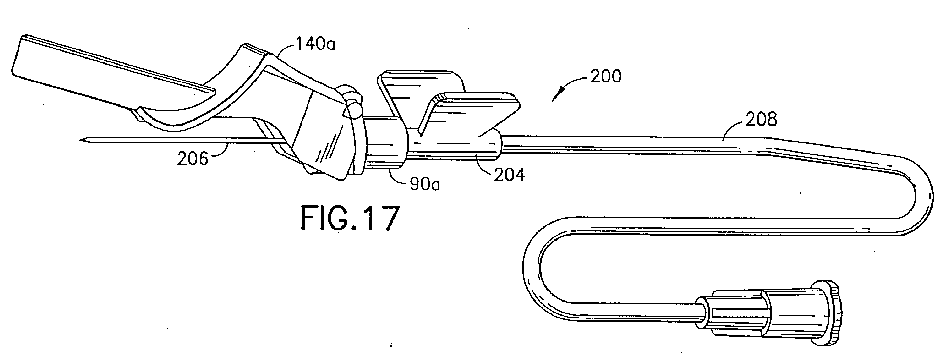

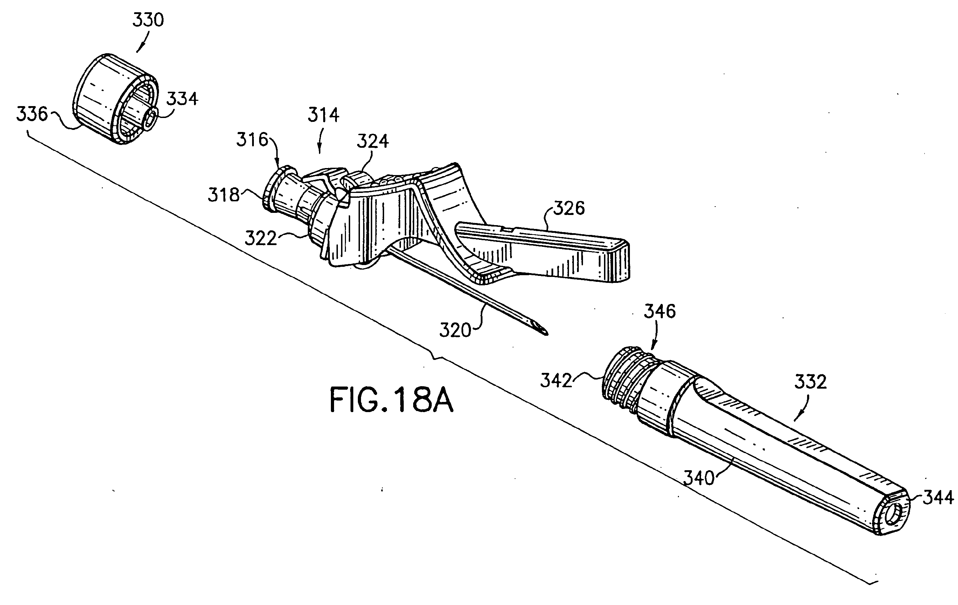

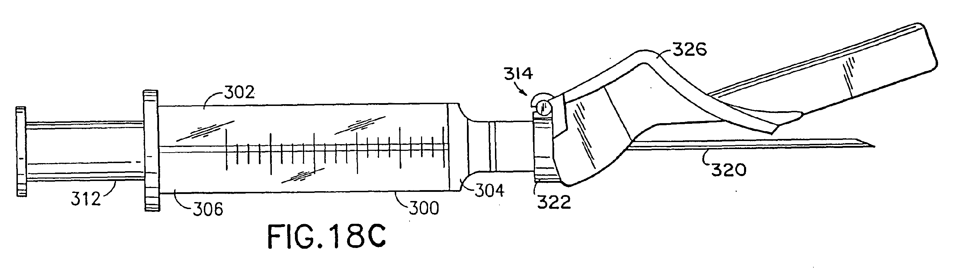

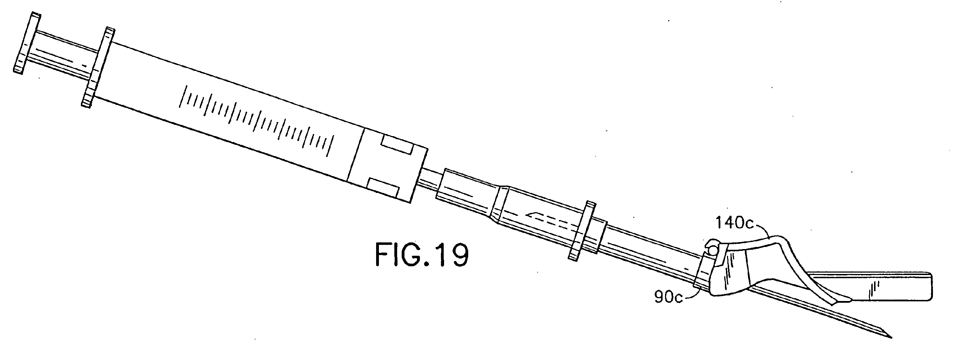

FIGS. 17, 18A-C, and 19 are further embodiments of the invention that may include components which are substantially identical to the components of FIGS. 1-3. Accordingly, similar components performing similar functions will be numbered identically to those components of FIGS. 1-3, except that a suffix "a" will be used to identify those similar components in FIG. 17, a suffix "b" will be used to identify those similar components in FIG. 18A-C and a suffix "c" will be used to identify those similar components in FIG. 19.

For purposes of illustration, hinged shield **140a** and collar **90a** are connected to a conventional IV infusion set, **200**, or butterfly structure comprising a needle body with a needle hub **204** extending from the forward end of the needle body and a needle **206** embedded in hub **204**. Extending from the rearward end of the needle body is flexible tubing **208** which is conventional and utilized to allow the user to manipulate the structure and to connect it subsequently to supplies of infusion liquids or for the return of collected blood if the arrangement is being used to collect blood.

Infusion set **200** further comprises flexible wings **210** attached to and projecting outwardly from needle hub **204**.

Alternatively, the safety shield assembly of the present invention may be used in conjunction with a syringe, as illustrated in FIGS. 18A-C.

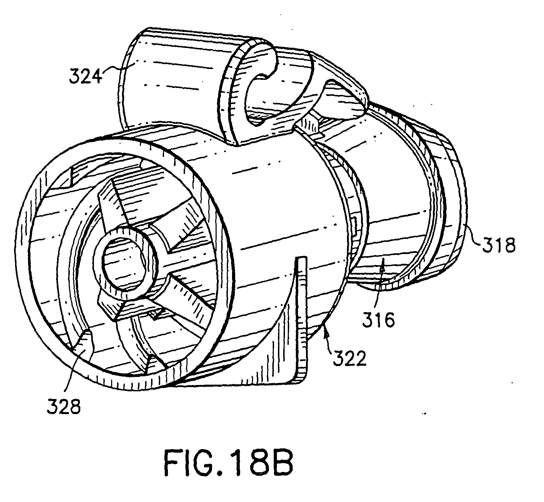

For purposes of illustration a conventional hypodermic syringe **300** comprises a syringe barrel **302** having a distal end **304**, a proximal end **306** and a plunger **312**. In this embodiment, a needle assembly **314** includes a hub **316** with a proximal end **318** that defines a female Luer fitting that can be mate with distal end **304** of syringe barrel **302**. An intravenous needle **320** projects distally from hub **316**. A collar **322** is mounted rigidly to hub **316** and includes a hook **324** at a location aligned substantially with the bevel up side of intravenous needle **320**. Alternatively, collar **322** and hub **316** may be a single component. A shield **326** is hingedly mounted to hook **324**. Collar **322** and hinged shield **326** are substantially identical to embodiments described and illustrated in greater detail above. In particular, collar **322** is provided with an array of internal threads **328**, as shown in FIG. 18B.

Pre-use sterility and safety are maintained by an end cap **330** and an IV shield **332**. End cap **330** includes a male Luer projection **334** and an outer collar **336**. Male Luer projection **334** is dimensioned to be frictionally retained within female Luer fitting at proximal end **318** of hub **316**. Outer collar **336** is dimensioned to be frictionally retained around hub **316**. End cap **330** can be removed from hub **316** with an exertion of proximally directed axially forces that may be combined with a slight rotational twisting force relative to hub **316**. End cap **330** prevents contamination of interior portions of hub **316**, and hence also prevents contamination of the lumen through intravenous needle **320**. IV shield **332** comprises a rigid generally tubular sidewall **340** with a proximal end **342** and a closed distal end **344**. Outer surface regions of IV shield **332** adjacent proximal end **342** define an array of external threads **346** that are dimensioned for threaded engagement with internal threads **328** on collar **322**. Thus, the IV shield can be threadedly mounted to collar **322** for protectively covering IV needle **320** and further contributing to sterility of IV needle **320**.

Prior to use, end cap **330** is mounted frictionally over proximal portions of needle hub **316** and IV shield **332** is mounted threadedly to internal threads **328** of collar **322** and over intravenous needle **320**. Hinged shield **326** then is rotated into a partly closed condition where proximal portions of hinged shield **322** partly surround and frictionally engage portions of IV shield **322** distally of and adjacent to external threads **346**.

The needle assembly is used by initially separating end cap **330** from needle hub **316**. Threaded engagement of IV shield **332** ensures that IV shield **332** will not inadvertently become separated from collar **322** in response to axial pulling forces exerted on end cap **330**. Thus, IV needle **320** remains safely covered and protected. Proximal end **318** of needle hub **316** then is mounted to distal end **304** of syringe **300**. IV shield **332** must be removed to access needle **320** and to use syringe **300**. The removal of IV shield **332** requires the disengagement of external threads **346** on IV shield **332** from internal threads **328** on collar **322**. However, the initial disposition of hinged shield **326** partly surrounding and adjacent IV shield **332** substantially prevents IV shield **332** from being threadedly disengaged from collar **322** without first rotating hinged shield **326** away from IV shield **332** and into the ready-to-use position. Thus, the user must follow the preferred practice of rotating hinged shield **326** away from needle cannula **320** and into the ready-to-use position prior to threadedly disengaging IV shield **332**. Accordingly, the needle assembly of FIGS. 18A-C substantially prevents the less safe practice of first removing IV shield **332** to expose needle **320** and then manually moving hinged shield **326** while the intravenous needle **320** is exposed. Furthermore, the needle assembly shown in FIGS. 18A-C ensures that the end cap **330** will be removed before exposing needle **320.** Accordingly, hub **316** is likely to be threadedly engaged with syringe **300** before rotating hinged shield **326** into the ready-to-use position and before separating IV shield **332**. FIGS. 18A-C show a threaded connection between IV shield **332** and collar **322**. However, other attachment mechanisms can be provided between IV shield **332** and collar **322** that would make separation difficult while hinged shield **326** is in partly surrounding disposition to IV shield **332**. For example, detents can be provided between IV shield **332** and collar **322** that would make simple pulling of IV shield **332** away from collar **322** difficult. The detent may require some rotational movement of IV shield relative to collar **322** to overcome frictional interference. Other such connections that would require secure gripping of IV shield **332** and/or twisting of IV shield **332** to effect removal may be provided.

Alternatively, the present invention may be used in conjunction with a catheter as illustrated in FIG. 19.

The shield and collar of the safety shield assembly of the present invention are comprised of moldable parts which can be mass produced from a variety of materials including, for example, polyethylene, polyvinyl chloride, polystyrene or polyethylene and the like. Materials will be selected which will provide the proper covering and support for the structure of the invention in its use, but which will provide also a degree of resiliency for the purpose of providing the cooperative movement relative to the shield and the collar of the assembly.

The illustrated embodiments show the first rigid sleeve or IV shield with external threads and the hub with the mating internal threads. However, the relative disposition of the internal and external threads may be reversed.

The illustrated embodiments show a cannula finger lock for engaging the needle. However, other means may be provided for maintaining the hinged shield around the needle, including more than one cannula finger lock or differently configured needle engaging structures.

## Claims

1. A medical implement comprising:
a hub having opposite proximal and distal ends;
a piercing element projecting from said distal end of said hub;
a protective cap removably mounted to said proximal end of said hub; and
an IV shield threadedly engaged with said distal end of said hub and protectively covering said piercing element, and a hinged shield hingedly mounted to said hub and selectively rotatable between a first position where said hinged shield lies substantially adjacent said IV shield, a second position where said hinged shield is rotated away from said IV shield and a third position where said hinged shield lockingly surrounds said piercing element, whereby said hinged shield must be rotated from said first position to said second position for threadedly disengaging said IV shield from said hub for exposing said piercing element.

2. The medical implement of claim 1, wherein said hub includes an array of internal threads, said IV shield comprising a proximal end with an array of external threads threadedly engaged with said internal threads of said hub.

3. The medical implement of claim 2 or 3, wherein said IV shield is a substantially tubular structure with a rigid sidewall extending distally beyond said piercing element.

4. The medical implement of any of claims 1-3, wherein said piercing element is a metallic needle cannula having a proximal end permanently mounted to said distal end of said hub and a sharply pointed distal end remote from said hub.

5. The medical implement of any of claims 1-4, wherein the protective cap is frictionally retained with said proximal end of said hub.

6. The medical implement of any of claims 1-5, wherein said proximal end of said hub defines a female Luer fitting, and wherein said cap comprises a male projection frictionally engaged with said female Luer fitting of said hub.

7. The medical implement of any of claims 1-4, wherein said protective cap is threadedly engaged with said proximal end of said hub.

8. The medical implement of any of claims 1-7, wherein said hinged shield comprises at least one resiliently deflectable lock for permanent locked engagement with said piercing element.

9. The medical implement of any of claims 1-8, wherein the hinged shield partly surrounds the IV shield when the hinge shield is in the first position.

10. The medical implement of any of claims 1-9, further comprising a holder for receiving an evacuated fluid collection tube, said holder being engageable with said hub.
